# EUROPEAN PATENT APPLICATION

(11) **EP 3 950 653 A1**
(43) Date of publication of application: **09.02.2022**
(21) Application number: 20777998.4
(22) Date of filing: 10.02.2020
(51) Int. Cl.: C07C 23/06, C07C 17/354, C07B 61/00, B01J 23/44

(54) **METHOD FOR PRODUCING HALOGENATED CYCLOALKANE COMPOUND**

(30) Priority: 27.03.2019 JP 2019061900
(71) Applicant: DAIKIN INDUSTRIES, LTD., Osaka-shi, Osaka 530-8323 (JP)
(72) Inventor: ETOU, Yuusuke, Osaka-shi, Osaka 530-8323 (JP); NAKAMURA, Shingo, Osaka-shi, Osaka 530-8323 (JP)
(74) Representative: Hoffmann Eitle
(86) International application number: PCT/JP2020/005154
(87) International publication number: WO 2020/195252

(57) **Abstract**

A halogenated cycloalkane compound is obtained at a high conversion rate by reacting a halogenated cycloalkene compound and a hydrogen-containing gas in the presence of a hydrogenated catalyst or a catalyst having a hydrogen content of 0.1 to 1.5 mass%.

## Description

### Technical Field

The present disclosure relates to a method for producing a halogenated cycloalkane compound.

### Background Art

Halogenated cycloalkane compounds are expected as dry etching gases for semiconductors, cleaning gases, and the like, and are saturated cyclic compounds having a halogen atom.

As a method for producing a halogenated cycloalkane compound, for example, in NPL 1, hexafluorocyclobutane (cC₄F₆H₂) is obtained from hexafluorocyclobutene (cC₄F₆) in the presence of a palladium catalyst supported on alumina.

### Citation List

### Patent Literature

NPL 1: Bulletin of the Academy of Sciences, 1962, pp. 2049-2051

### Summary of Invention

### Technical Problem

An object of the present disclosure is to provide a method that can obtain a halogenated cycloalkane compound at a high conversion rate.

### Solution to Problem

The present disclosure includes the following configurations.

Item 1. A method for producing a halogenated cycloalkane compound, comprising:
(IIa) reacting a halogenated cycloalkene compound and a hydrogen-containing gas in the presence of a hydrogenated catalyst to obtain a halogenated cycloalkane compound.

Item 2. The production method according to Item 1, comprising, before step (IIa):
(I) bringing a catalyst into contact with a hydrogen-containing gas to obtain the hydrogenated catalyst.

Item 3. A method for producing a halogenated cycloalkane compound, comprising:
(IIb) reacting a halogenated cycloalkene compound and a hydrogen-containing gas in the presence of a catalyst having a hydrogen content of 0.1 to 1.5 mass% to obtain a halogenated cycloalkane compound.

Item 4. The production method according to Item 3, wherein the catalyst has a specific surface area of 50 to 500 m²/g.

Item 5. The production method according to any one of Items 1 to 4, wherein the halogenated cycloalkane compound is a halogenated cycloalkane compound represented by formula (1): 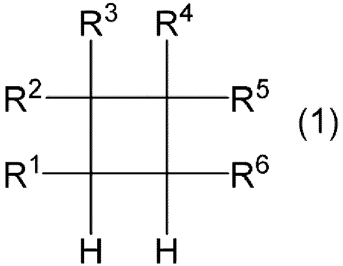 wherein R¹, R², R³, R⁴, R⁵, and R⁶ are the same or different and each is a hydrogen atom, a halogen atom, or a perfluoroalkyl group; when cis-trans isomers are present, both isomers are contained; provided that not all of R¹, R², R³, R⁴, R⁵, and R⁶ are hydrogen atoms.

Item 6. The production method according to any one of Items 1 to 5, wherein the halogenated cycloalkene compound is a halogenated cycloalkene compound represented by formula (2): 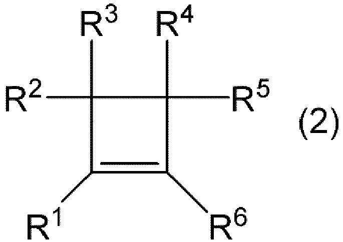 wherein R¹, R², R³, R⁴, R⁵, and R⁶ are the same or different and each is a hydrogen atom, a halogen atom, or a perfluoroalkyl group; when cis-trans isomers are present, both isomers are contained; provided that not all of R¹, R², R³, R⁴, R⁵, and R⁶ are hydrogen atoms.

Item 7. The production method according to any one of Items 1 to 6, wherein the catalyst and hydrogenated catalyst comprise at least one transition metal element belonging to groups 8 to 11 of the periodic table.

Item 8. The production method according to any one of Items 2 and 4 to 7, wherein step (I) is a step of bringing 10 mL to 1000 L of the hydrogen-containing gas into contact with 1 mol of the catalyst.

Item 9. The production method according to any one of Items 2 and 4 to 8, wherein in step (I), the contact time (W/F) of the hydrogen-containing gas with the catalyst is 0.1 to 200 g·sec/cc.

Item 10. A hydrogenated catalyst for use in obtaining a halogenated cycloalkane compound by reacting a halogenated cycloalkene compound and a hydrogen-containing gas, the hydrogenated catalyst having a hydrogen content of 0.1 to 1.5 mass%.

Item 11. The hydrogenated catalyst according to Item 10, comprising at least one transition metal element belonging to groups 8 to 11 of the periodic table.

Item 12. The hydrogenated catalyst according to Item 10 or 11, which has a specific surface area of 50 to 500 m²/g.

Item 13. A composition comprising a halogenated cycloalkane compound represented by formula (1): 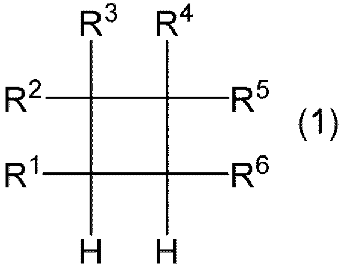 wherein R¹, R², R³, R⁴, R⁵, and R⁶ are the same or different and each is a hydrogen atom, a halogen atom, or a perfluoroalkyl group; when cis-trans isomers are present, both isomers are contained; provided that not all of R¹, R², R³, R⁴, R⁵, and R⁶ are hydrogen atoms,
the halogenated cycloalkane compound represented by formula (1) being contained in an amount of 90.0 to 99.9 mol% based on the total amount of the composition, which is taken as 100 mol%.

Item 14. The composition according to Item 13, which is used as a cleaning gas or an etching gas.

### Advantageous Effects of Invention

According to the present disclosure, a halogenated cycloalkane compound can be obtained at a high conversion rate.

### Description of Embodiments

In the present specification, the term "comprise" is a concept including "comprising," "consisting essentially of," and "consisting of." In the present specification, the numerical range indicated by "A to B" means A or more and B or less.

In the present disclosure, "selectivity" means the ratio (mol%) of the total molar amount of the target compound contained in the effluent gas from the reactor outlet, based on the total molar amount of compounds other than the raw material compound in the effluent gas.

In the present disclosure, "conversion rate" means the ratio (mol%) of the total molar amount of compounds other than the raw material compound contained in the effluent gas from the reactor outlet, based on the molar amount of the raw material compound supplied to the reactor.

In the present disclosure, "hydrogenated catalyst" refers to a catalyst that has been hydrogenated.

### 1. Hydrogenated Catalyst

In the present disclosure, the hydrogenated catalyst is used to react a halogenated cycloalkene compound and a hydrogen-containing gas to obtain a halogenated cycloalkane compound, and is obtained by bringing a catalyst into contact with a hydrogen-containing gas. Further, such a hydrogenated catalyst has a hydrogen content of 0.1 to 1.5 mass%. The catalyst conventionally used in NPL 1 for the above reaction was not hydrogenated (did not contain hydrogen), and the hydrogen content was less than 0.1 mass%. Accordingly, when a halogenated cycloalkene compound as a substrate was brought into contact with a hydrogen-containing gas, the catalyst reacted with the hydrogen-containing gas, and the ratio of the hydrogen-containing gas to react with the substrate decreased, thereby reducing the conversion rate. For example, in NPL 1, about 14% of unreacted raw materials remained, and the conversion rate was about 86%. In the present disclosure, due to the use of a hydrogenated catalyst, when a halogenated cycloalkene compound as a substrate is brought into contact with a hydrogen-containing gas, the reaction between the hydrogenated catalyst and the hydrogen-containing gas can be suppressed; that is, it is possible to suppress the consumption of hydrogen in the hydrogen-containing gas by the catalyst, and the substrate and hydrogen in the hydrogen-containing gas can react efficiently. Accordingly, a halogenated cycloalkane compound can be obtained at a high conversion rate and, in some cases, a high yield and a high selectivity by reacting the halogenated cycloalkene compound and the hydrogen-containing gas.

The hydrogenated catalyst of the present disclosure described above can be produced by hydrogenating a catalyst.

The metal species that constitute the catalyst are preferably transition metal elements belonging to groups 8 to 11 of the periodic table, and more preferably transition metal elements belonging to groups 9 to 10 of the periodic table. Specific examples of such metal species include platinum, palladium, rhodium, nickel, and the like. The hydrogenated catalyst of the present disclosure may contain such a metal alone, may be a porous metal catalyst, or may contain such a metal as a compound with other elements. Usable examples include catalysts in which non-catalytic metal species, such as aluminum, silicon, magnesium, and zinc, are eluted from alloys of catalytic metal species, such as platinum, palladium, rhodium, and nickel, and the non-catalytic metal species using an acid or alkaline solution (Raney catalysts), Pt(PtO₂), Adams' catalyst (PtO₂-H₂O), colloidal palladium, colloidal platinum, platinum black, and the like. These can be used singly or in combination of two or more.

Further, in the present disclosure, the metal species mentioned above can be directly used as catalysts, or can be supported on a carrier for use. The carrier that can be used in this case is not limited, and examples include carbon, alumina (Al₂O₃), zirconia (ZrO₂), silica (SiO₂), titania (TiO₂), and the like. Of these, carbon, alumina, etc. are preferred, and alumina is more preferred, from the viewpoint of conversion rate, yield, and selectivity in the production of a halogenated cycloalkane compound by the production method described later.

The hydrogenated catalyst of the present disclosure can be obtained by bringing the catalyst mentioned above into contact with a hydrogen-containing gas. The catalyst before hydrogenation is not limited, and can be a hydrogen-free catalyst.

Examples of the hydrogen-containing gas include a hydrogen gas, as well as mixtures of a hydrogen gas and other gases (e.g., a mixed gas of hydrogen and an inert gas such as nitrogen or argon mixed at any ratio, and oxyhydrogen, which is a mixed gas of oxygen and hydrogen). From the viewpoint of the efficiency of hydrogenation of the catalyst, it is preferable to use a hydrogen gas. These hydrogen-containing gases can be used singly or in combination of two or more.

When the catalyst and the hydrogen-containing gas are brought into contact with each other, it is preferable to bring 10 mL to 1000 L (particularly 100 mL to 100 L) of the hydrogen-containing gas into contact with 1 mol of the catalyst. Within these ranges, the catalyst can be hydrogenated more moderately, and it is possible to further improve the conversion rate, yield, and selectivity in the production of a halogenated cycloalkane compound by the production method described later.

When the catalyst and the hydrogen-containing gas are brought into contact with each other, the contact temperature (when using a gas-phase reaction (particularly a gas-phase flow reaction), the temperature of a system for flow) is preferably 50 to 400°C (particularly 100 to 300°C) . Within these ranges, the catalyst can be hydrogenated more moderately, and it is possible to further improve the conversion rate, yield, and selectivity in the production of a halogenated cycloalkane compound by the production method described later.

When the catalyst and the hydrogen-containing gas are brought into contact with each other, the contact time of the hydrogen-containing gas with the catalyst (W/F) [W: weight (g) of catalyst, F: flow rate (cc/sec) of hydrogen-containing gas] when using a gas-phase reaction (particularly a gas-phase flow reaction) is preferably 0.1 to 200 g·sec/cc min (particularly 1 to 100 g·sec/cc). Within these ranges, the catalyst can be hydrogenated more moderately, and it is possible to further improve the conversion rate, yield, and selectivity in the production of a halogenated cycloalkane compound by the production method described later.

The reaction pressure for reacting the catalyst and the hydrogen-containing gas in the present disclosure is preferably - 50 kPa to 10 MPa, and more preferably 10 kPa to 5 MPa. Within these ranges, the catalyst can be hydrogenated more moderately, and it is possible to further improve the conversion rate, yield, and selectivity in the production of a halogenated cycloalkane compound by the production method described later. In the present disclosure, the pressure is gauge pressure unless otherwise specified.

The hydrogenation of a catalyst can be carried out in this manner. The resulting hydrogenated catalyst has a hydrogen content of 0.1 to 1.5 mass%.

The hydrogen content of the hydrogenated catalyst is 0.1 to 1.5 mass%, and preferably 0.2 to 1.0 mass%, based on the total mass of the hydrogenated catalyst, which is taken as 100 mass%. If the hydrogen content is less than 0.1 mass%, in the production of a halogenated cycloalkane compound by the production method described later, the reaction cannot proceed efficiently, and the conversion rate and yield decrease. On the other hand, the hydrogen content is preferably 1.5 mass% or less, from the viewpoint of facilitating the suppression of excessive reaction in the production of a halogenated cycloalkane compound by the production method described later, and from the viewpoint that it is easy to suppress the formation of compounds in which the halogen atom contained in the halogenated cycloalkane compound is replaced by a hydrogen atom, and it is easy to further improve the yield of the target product. The hydrogen content of the hydrogenated catalyst is measured by measuring the catalyst weight before and after hydrogenation.

The specific surface area of the hydrogenated catalyst is preferably 50 m²/g to 500 m²/g, more preferably 75 to 475 m²/g, and even more preferably 100 to 450 m²/g. When the specific surface area is within these ranges, in the production of a halogenated cycloalkane compound by the production method described later, the reaction can proceed more efficiently, and the conversion rate and yield can be further improved. On the other hand, it is possible to further suppress excessive progress of the reaction in the production of a halogenated cycloalkane compound by the production method described later, and it is possible to more effectively suppress the generation of compounds in which the halogen atom contained in the halogenated cycloalkane compound is replaced by a hydrogen atom, and the decrease in the yield of the target product. The specific surface area of the hydrogenated catalyst is measured by the gas adsorption method.

### 2. Method for Producing Halogenated Cycloalkane Compound

The method for producing a halogenated cycloalkane compound of the present disclosure comprises (IIa) reacting a halogenated cycloalkene compound and a hydrogen-containing gas in the presence of a hydrogenated catalyst to obtain a halogenated cycloalkane compound.

The hydrogenated catalyst used in step (IIa) can be obtained by (I) bringing a catalyst into contact with a hydrogen-containing gas to obtain the hydrogenated catalyst.

Regarding step (I) and the hydrogenated catalyst, those explained in "1. Hydrogenated Catalyst" above can be used. That is, using a catalyst having a hydrogen content of 0.1 to 1.5 mass% in place of the hydrogenated catalyst, the production method can comprise (IIb) reacting a halogenated cycloalkene compound and a hydrogen-containing gas in the presence of the catalyst having a hydrogen content of 0.1 to 1.5 mass% to obtain a halogenated cycloalkane compound.

In the reactions of steps (IIa) and (IIb), conventionally used catalysts were not hydrogenated (did not contain hydrogen); thus, when a halogenated cycloalkene compound as a substrate was brought into contact with a hydrogen-containing gas, the catalyst reacted with the hydrogen-containing gas, and the ratio of the hydrogen-containing gas to react with the substrate decreased, thereby reducing the conversion rate. In the present disclosure, due to the use of a catalyst that has been hydrogenated in advance, when a halogenated cycloalkene compound as a substrate is brought into contact with a hydrogen-containing gas, the reaction between the hydrogenated catalyst and the hydrogen-containing gas can be suppressed, and a halogenated cycloalkane compound can be obtained at a high conversion rate and, in some cases, a high yield and selectivity by reacting the halogenated cycloalkene compound and the hydrogen-containing gas.

The halogenated cycloalkene compound as a substrate that can be used in steps (IIa) and (IIb) is not limited. Examples include a halogenated cycloalkene compound represented by formula (2): 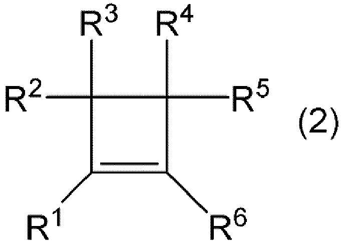 wherein R¹, R², R³, R⁴, R⁵, and R⁶ are the same or different and each is a hydrogen atom, a halogen atom, or a perfluoroalkyl group; when cis-trans isomers are present, both isomers are contained; provided that not all of R¹, R², R³, R⁴, R⁵, and R⁶ are hydrogen atoms.

In formula (2), examples of the halogen atom represented by R¹, R², R³, R⁴, R⁵, and R⁶ include a fluorine atom, a chlorine atom, a bromine atom, and an iodine atom.

In formula (2), the perfluoroalkyl group represented by R¹, R², R³, R⁴, R⁵, and R⁶ refers to an alkyl group in which all of the hydrogen atoms are replaced by fluorine atoms. Such a perfluoroalkyl group is, for example, a perfluoroalkyl group having 1 to 20 carbon atoms, preferably 1 to 12 carbon atoms, more preferably 1 to 6 carbon atoms, even more preferably 1 to 4 carbon atoms, and particularly preferably 1 to 3 carbon atoms. The perfluoroalkyl group is preferably a linear or branched perfluoroalkyl group. Such a perfluoroalkyl group is preferably, for example, a trifluoromethyl group (CF₃-) or a pentafluoroethyl group (C₂F₅-).

In the halogenated cycloalkene compound, which is a substrate, all of R¹, R², R³, R⁴, R⁵, and R⁶ are preferably fluorine atoms, perfluoroalkyl groups, or the like, and more preferably fluorine atoms, from the viewpoint that a halogenated cycloalkane compound containing a fluorine atom can be produced particularly at a high conversion rate, yield, and selectivity.

R¹, R², R³, R⁴, R⁵, and R⁶ may be the same or different.

Specific examples of the halogenated cycloalkene compound as a substrate that satisfies the above conditions include the following:

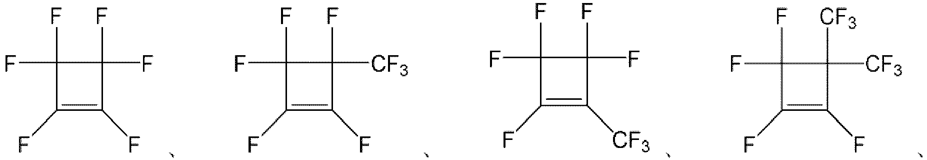

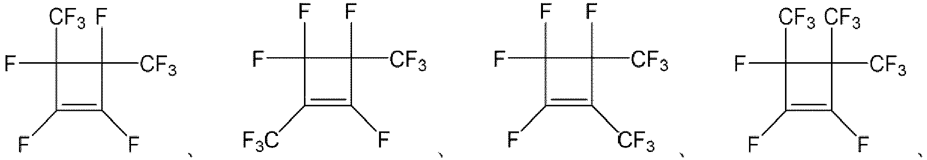

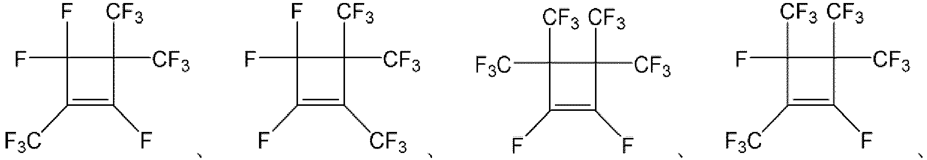

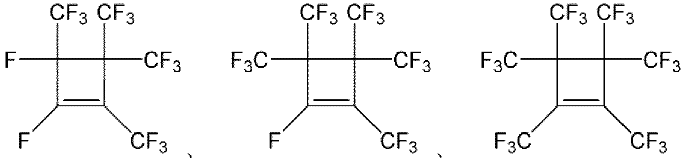

These halogenated cycloalkene compounds can be used singly or in combination of two or more. Such halogenated cycloalkene compounds may be known or commercially available products.

Examples of the hydrogen-containing gas to react with the halogenated cycloalkene compound include a hydrogen gas, as well as mixtures of a hydrogen gas and other gases (e.g., a mixed gas of hydrogen and an inert gas such as nitrogen or argon mixed at any ratio, and oxyhydrogen, which is a mixed gas of oxygen and hydrogen). However, the reactions in steps (IIa) and (IIb) of the present disclosure are hydrogen addition reactions, as described later, and it is thus preferable that the hydrogen-containing gas does not contain hydrogen halide (hydrogen fluoride) etc., or contains a very small amount (e.g., 5% by volume or less based on the total amount of the hydrogen-containing gas). From the viewpoint of the reaction conversion rate, yield, and selectivity, it is preferable to use a hydrogen gas. These hydrogen-containing gases can be used singly or in combination of two or more.

The hydrogen-containing gas is generally preferably supplied in the gas-phase state to a reactor together with the halogenated cycloalkene compound (substrate). The amount of the hydrogen-containing gas supplied is about 1.0 to 30.0 mol, particularly 1.0 to 20.0 mol, further about 1.0 to 2.5 mol, and particularly about 1.3 to 2.0 mol, per mol of the halogenated cycloalkene compound (substrate). Within these ranges, the addition reaction with the hydrogen-containing gas can proceed better, the formation of impurities can be further reduced, and the resulting product has a high selectivity of a halogenated cycloalkane compound, which can be recovered in high yield.

In steps (IIa) and (IIb) of the present disclosure, the hydrogenated catalysts described above are used as catalysts. The reaction between a halogenated cycloalkene compound and a hydrogen-containing gas in step (II) of the present disclosure is the addition reaction of hydrogen by the hydrogen-containing gas, and can be carried out in the gas phase in the presence of a hydrogenated catalyst. The step of reacting a halogenated cycloalkene compound and a hydrogen-containing gas (addition reaction) in the present disclosure is preferably carried out in the gas phase, particularly by a gas-phase continuous flow process using a fixed bed reactor. When a gas-phase continuous flow process is used, the device, operation, etc. can be simplified, and it is economically advantageous.

In steps (IIa) and (IIb) of reacting a halogenated cycloalkene compound and a hydrogen-containing gas in the present disclosure, the lower limit of the reaction temperature is generally preferably 20°C or higher, more preferably 30°C or higher, and even more preferably 40°C or higher, from the viewpoint that the addition reaction with the hydrogen-containing gas can proceed more efficiently to further improve the conversion rate, and the target compound can be obtained with higher selectivity.

The upper limit of the reaction temperature for reacting a halogenated cycloalkene compound and a hydrogen-containing gas in the present disclosure is generally preferably 500°C or lower, more preferably 400°C or lower, and even more preferably 300°C or lower, from the viewpoint of more efficiently promoting the addition reaction with the hydrogen-containing gas to further improve the conversion rate, and obtaining the target compound with higher selectivity, and from the viewpoint of further suppressing the decrease in selectivity due to decomposition or polymerization of the reaction product.

Regarding the reaction time for reacting a halogenated cycloalkene compound and a hydrogen-containing gas in the present disclosure, for example, when using a gas-phase reaction (particularly a gas-phase flow reaction), the contact time of the raw material compound with the catalyst (W/F) [W: weight (g) of catalyst, F: flow rate (cc/sec) of raw material compound] is preferably 5.0 to 200 g·sec/cc, more preferably 6.0 to 150 g·sec/cc, and even more preferably 7.0 to 100 g·sec/cc, from the viewpoint that the conversion rate of the reaction is particularly high and a perfluorocycloalkene compound can be obtained with higher yield and higher selectivity. The above-mentioned W/F specifies the reaction time particularly when using a gas-phase flow reaction; however, even when using a batch reaction, the contact time can be appropriately set. The contact time refers to the time of contact between the substrate and the hydrogenated catalyst.

The reaction pressure for reacting a halogenated cycloalkene compound and a hydrogen-containing gas in the present disclosure is preferably -0.05 MPa to 2 MPa, more preferably - 0.01 MPa to 1 MPa, and even more preferably normal pressure to 0.5 MPa, in terms of promoting the addition reaction with the hydrogen-containing gas more efficiently. In the present disclosure, the pressure is gauge pressure unless otherwise specified.

In the reaction between a halogenated cycloalkene compound and a hydrogen-containing gas in the present disclosure, the reactor for reacting the substrate and the hydrogenated catalyst by bringing them into contact with each other is not limited in its shape and structure as long as it can withstand the temperature and pressure described above. Examples of the reactor include a vertical reactor, a horizontal reactor, a multitubular reactor, and the like. Examples of the material of the reactor include glass, stainless steel, iron, nickel, iron-nickel alloys, and the like.

The reaction between a halogenated cycloalkene compound and a hydrogen-containing gas (addition reaction with the hydrogen-containing gas) in the present disclosure can be carried out in a batch mode or a flow mode in which the substrate is continuously fed into the reactor and the target compound is continuously withdrawn from the reactor. If the target compound remains in the reactor, the elimination reaction can proceed further; thus, it is preferable to carry out the reaction in a flow mode. Steps (IIa) and (IIb) of reacting a halogenated cycloalkene compound and a hydrogen-containing gas in the present disclosure are preferably carried out in the gas phase, particularly by a gas-phase continuous flow process using a fixed bed reactor. When a gas-phase continuous flow process is used, the device, operation, etc. can be simplified, and it is economically advantageous.

The atmosphere when reacting a halogenated cycloalkene compound and a hydrogen-containing gas in the present disclosure is preferably an inert gas atmosphere, a hydrogen gas atmosphere, or the like, from the viewpoint of suppressing the deterioration of the hydrogenated catalyst. Examples of the inert gas include nitrogen, helium, argon, and the like. Among these inert gases, nitrogen is preferable from the viewpoint of cost reduction. The concentration of the inert gas is preferably 0 to 50 mol% of the gas component introduced into the reactor.

Examples of the target compound of the present disclosure obtained in this manner include a halogenated cycloalkane compound represented by formula (1): 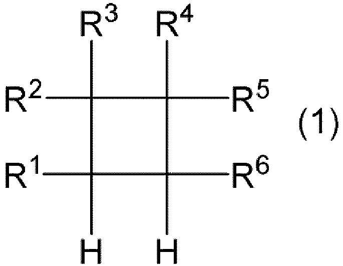 wherein R¹, R², R³, R⁴, R⁵, and R⁶ are the same or different and each is a hydrogen atom, a halogen atom, or a perfluoroalkyl group; when cis-trans isomers are present, both isomers are contained; provided that not all of R¹, R², R³, R⁴, R⁵, and R⁶ are hydrogen atoms.

R¹, R², R³, R⁴, R⁵, and R⁶ in formula (1) correspond to R¹, R², R³, R⁴, R⁵, and R⁶ in formula (2), respectively. Therefore, examples of the halogenated cycloalkane compound represented by formula (1) to be produced include the following:

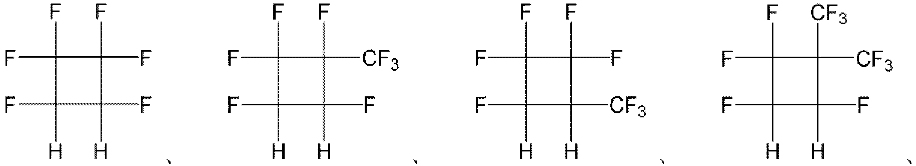

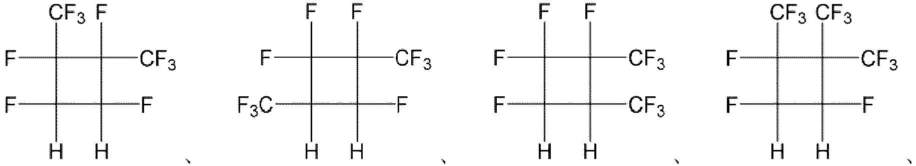

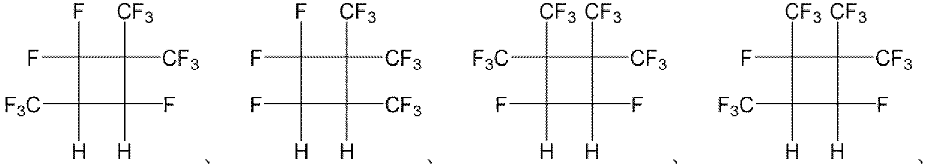

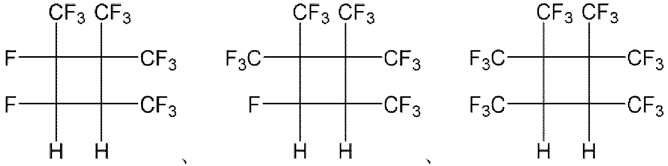

After the reaction between the halogenated cycloalkene compound and the hydrogen-containing gas (addition reaction with the hydrogen-containing gas) is completed, purification can be optionally performed according to a conventional method, thereby obtaining a halogenated cycloalkane compound, which is the target compound. According to the production method of the present invention, the halogenated cycloalkane compound produced can contain both cis- and trans-isomers, and may be obtained as a mixture of cis- and trans-isomers; however, they can be isolated by purification according to a conventional method. When the reaction between a halogenated cycloalkene compound and a hydrogen-containing gas is allowed to proceed without hydrogenation of the catalyst in advance as in the conventional case, hydrogenation of the catalyst, which is an exothermic reaction, also occurs at the same time, the heat of this reaction raises the reaction temperature locally, and the cis-isomer, which is structurally difficult to generate, is likely to be generated. On the other hand, according to the present disclosure, due to the use of a catalyst that has been hydrogenated in advance or a catalyst with a high hydrogen content, such an exothermic reaction is suppressed, the cis-isomer, which is structurally difficult to generate, is less likely to be generated, and the trans-isomer is selectively generated. However, since the cis-isomer of the halogenated cycloalkane compound and the trans-isomer of the halogenated cycloalkane compound do not have a large difference in etching performance, they can be directly used without isolation as an etching gas or a cleaning gas.

### 2. Halogenated Cycloalkane Composition

The halogenated cycloalkane compound can be obtained in the above manner, and may be obtained in the form of a halogenated cycloalkane composition comprising the halogenated cycloalkane compound and a halogenated cycloalkene compound, which is a substrate. Further, the halogenated cycloalkane compound may be obtained as a mixture of the cis-isomer of the halogenated cycloalkane compound and the trans-isomer of the halogenated cycloalkane compound (in this case, the trans-isomer is often obtained selectively). Therefore, the halogenated cycloalkane compositions of the present invention include compositions comprising the cis-isomer of the halogenated cycloalkane compound and the trans-isomer of the halogenated cycloalkane compound.

In the halogenated cycloalkane composition of the present disclosure, the amount of the halogenated cycloalkane compound represented by formula (1) is preferably 90.0 to 99.9 mol%, more preferably 91.0 to 99.8 mol%, even more preferably 92.0 to 99.7 mol%, and particularly preferably 95.0 to 99.6 mol%, when the total amount of the halogenated cycloalkane composition of the present disclosure is taken as 100 mol%. The amount of the halogenated cycloalkane compound represented by formula (1) means the total amount of the cis-isomer of the halogenated cycloalkane compound and the trans-isomer of the halogenated cycloalkane compound.

According to the production method of the present disclosure, regarding the halogenated cycloalkane compound, the trans-isomer is more often obtained selectively than the cis-isomer, as described above. Therefore, when the total amount of the halogenated cycloalkane composition of the present disclosure is taken as 100 mol%, the amount of the cis-isomer of the halogenated cycloalkane compound is preferably 5.0 to 20.0 mol% (particularly 7.0 to 18.0 mol%), and the amount of the trans-isomer of the halogenated cycloalkane compound is preferably 78.0 to 95.0 mol% (particularly 80.0 to 93.0 mol%).

According to the production method of the present disclosure, even when being obtained as a halogenated cycloalkane composition, the halogenated cycloalkane compound represented by formula (1) can be obtained at a high reaction conversion rate and, in some cases, a high yield and a high selectivity, as described above. Therefore, it is possible to reduce components other than the halogenated cycloalkane compound represented by formula (1) in the halogenated cycloalkane composition, which can reduce the purification work to obtain the halogenated cycloalkane compound represented by formula (1).

The halogenated cycloalkane composition of the present disclosure described above can be effectively used for various applications, such as etching gases for forming cutting-edge microstructures in semiconductors, liquid crystals, etc., as well as cleaning gases, as in the case of the halogenated cycloalkane compound alone.

Although the embodiments are described above, various changes in form and details can be made without departing from the spirit and scope of the claims.

### Examples

The features of the present disclosure are clarified below while showing Examples. The present disclosure is not limited to these Examples.

In the methods for producing a halogenated cycloalkane compound of Examples 3 to 8 and Comparative Example 1, the raw material compound was a halogenated cycloalkene compound represented by formula (2) wherein R¹, R², R³, R⁴, R⁵, and R⁶ are fluorine atoms, and a halogenated cycloalkane compound was obtained by a hydrogen addition reaction according to the following reaction formula:

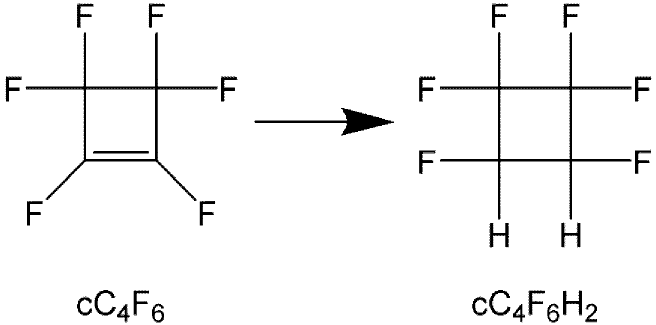

### Example 1: Production of Hydrogenated Pd/C Catalyst

A SUS pipe (outer diameter: 1/2 inch) was used as a reaction tube and filled with, as a catalyst before hydrogenation, 5.0 g of a Pd/C catalyst in which palladium was supported on carbon (produced by N.E. Chemcat Corporation, containing 3 mass% of palladium based on the catalyst mass).

A hydrogen gas was allowed to flow through the reaction tube at a flow rate of 100 ccm, and hydrogenation was carried out at a reaction tube temperature of 200°C for 60 minutes. That is, the contact time was adjusted so that W/F (catalyst weight (g)/hydrogen flow rate (cc/min)) of the hydrogen gas to be brought into contact with the Pd/C catalyst was 3. The resulting hydrogenated Pd/C catalyst was then extracted and used for the hydrogen addition reaction. The specific surface area of the resulting hydrogenated Pd/C catalyst measured by the gas adsorption method was 354 m²/g, and the hydrogen content measured by the weight difference before and after the reaction was 0.8 mass%.

### Example 2: Production of Hydrogenated Pd/Al₂O₃ Catalyst

A hydrogenated Pd/Al₂O₃ catalyst was obtained in the same manner as in Example 1, except that a Pd/Al₂O₃ catalyst in which palladium was supported on alumina (produced by N.E. Chemcat Corporation, containing 0.5 mass% of palladium based on the catalyst mass) was used as the catalyst before hydrogenation in place of the Pd/C catalyst in which palladium was supported on carbon. The specific surface area of the resulting hydrogenated Pd/C catalyst measured by the gas adsorption method was 234 m²/g, and the hydrogen content measured by the weight difference before and after the reaction was 0.7 mass%.

### Examples 3 to 6: Hydrogen Addition Reaction Using Hydrogenated Pd/C Catalyst

5.0 g of the hydrogenated Pd/C catalyst obtained in Example 1 was added as a catalyst to a SUS pipe (outer diameter: 1/2 inch), which was a reaction tube. After drying at 200°C for 2 hours in a nitrogen atmosphere, cC₄F₆H₂ (substrate) and a hydrogen gas were allowed to flow through the reaction tube at normal pressure so that the contact time (W/F) of cC₄F₆H₂ (substrate) and the hydrogenated Pd/C catalyst was 8.6 g·sec/cc, 13.0 g·sec/cc, 15.0 g·sec/cc, or 18.0 g·sec/cc.

The reaction was allowed to proceed by a gas-phase continuous flow process.

The reaction tube was heated at 60°C, 100°C, or 165°C to start the hydrogen addition reaction.

The contact time was adjusted so that the molar ratio (H_{2/}cC₄F₆H₂ ratio) of the hydrogen gas to be brought into contact with cC₄F₆H₂ (substrate) was 1.1, 1.5, or 2.0, and the distillate that passed through the abatement tower was collected one hour after the start of the reaction.

After that, mass spectrometry was performed by gas chromatography/mass spectrometry (GC/MS) using a gas chromatograph (produced by Shimadzu Corporation, trade name: "GC-2014"). Structural analysis using NMR spectra was performed using NMR (produced by JEOL Ltd., trade name: "400YH").

From the results of mass spectrometry and structural analysis, it was confirmed that cC₄F₆H₂ was produced as a target compound. In Example 3, the conversion rate from cC₄F₆H₂ (substrate) was 100.0 mol%, and the selectivity of cC₄F₆H₂ (target compound) was 99.6 mol% (cis-isomer: 17.7 mol%, trans-isomer: 81.9 mol%). In Example 4, the conversion rate from cC₄F₆H₂ (substrate) was 97.6 mol%, and the selectivity of cC₄F₆H₂ (target compound) was 99.0 mol% (cis-isomer: 17.1 mol%, trans-isomer: 81.9 mol%). In Example 5, the conversion rate from cC₄F₆H₂ (substrate) was 94.2 mol%, and the selectivity of cC₄F₆H₂ (target compound) was 97.7 mol% (cis-isomer: 15.9 mol%, trans-isomer: 81.8 mol%). In Example 6, the conversion rate from cC₄F₆H₂ (substrate) was 99.8 mol%, and the selectivity of cC₄F₆H₂ (target compound) was 98.9 mol% (cis-isomer: 7.2 mol%, trans-isomer: 91.7 mol%).

### Examples 7 and 8: Hydrogen Addition Reaction Using Hydrogenated Pd/Al₂O₃ Catalyst

The reaction was allowed to proceed in the same manner as in Examples 3 to 6, except that the hydrogenated Pd/Al₂O₃ catalyst obtained in Example 2 was used as the catalyst, the reaction temperature was 60°C or 100°C, the contact time (W/F) of cC₄F₆H₂ (substrate) and the hydrogenated Pd/Al₂O₃ catalyst was 8.6 g·sec/cc or 15.0 g·sec/cc, and the contact time was adjusted so that the molar ratio (H_{2/}cC₄F₆H₂ ratio) of the hydrogen gas to be brought into contact with cC₄F₆H₂ (substrate) was 1.5.

From the results of mass spectrometry and structural analysis, it was confirmed that cC₄F₆H₂ was produced as a target compound. In Example 7, the conversion rate from cC₄F₆H₂ (substrate) was 98.6 mol%, and the selectivity of cC₄F₆H₂ (target compound) was 99.7 mol% (cis-isomer: 17.5 mol%, trans-isomer: 82.2 mol%). In Example 8, the conversion rate from cC₄F₆H₂ (substrate) was 99.8 mol%, and the selectivity of cC₄F₆H₂ (target compound) was 99.4 mol% (cis-isomer: 7.4 mol%, trans-isomer: 92.0 mol%).

### Comparative Example 1: Hydrogen Addition Reaction Using Pd/C Catalyst (Without Hydrogenation)

The reaction was allowed to proceed in the same manner as in Examples 3 to 6, except that a non-hydrogenated Pd/C catalyst (produced by N.E. Chemcat Corporation, containing 5 mass% of palladium based on the catalyst mass; specific surface area: 410 m²/g; not containing hydrogen) was used as the catalyst, the reaction temperature was 63.4°C, the contact time (W/F) of cC₄F₆H₂ (substrate) and the hydrogenated Pd/Al₂O₃ catalyst was 18.0 g·sec/cc, and the contact time was adjusted so that the molar ratio (H_{2/}cC₄F₆H₂ ratio) of the hydrogen gas to be brought into contact with cC₄F₆H₂ (substrate) was 1.5.

From the results of mass spectrometry and structural analysis, it was confirmed that cC₄F₆H₂ was produced as a target compound. However, the conversion rate from cC₄F₆H₂ (substrate) was 86.3 mol%, which was not sufficient, and the selectivity of cC₄F₆H₂ (target compound) was 98.2 mol% (cis-isomer: 20.9 mol%, trans-isomer: 77.3 mol%).

The results are shown in Table 1.

**Table 1**

| | | | Example | | | | | | Comparative Example |
|---|---|---|---|---|---|---|---|---|---|
| | | | 3 | 4 | 5 | 6 | 7 | 8 | 1 |
| Catalyst | | | Hydrogenated Pd/C | Hydrogenated Pd/C | Hydrogenated Pd/C | Hydrogenated Pd/C | Hydrogenated Pd/Al₂O₃ | Hydrogenated Pd/Al₂O₃ | Pd/C |
| Temperature (°C) | | | 60 | 60 | 165 | 100 | 60 | 100 | 63.4 |
| W/F (g·sec/cc) | | | 13.0 | 15.0 | 18.0 | 8.6 | 15.0 | 8.6 | 18.0 |
| Molar ratio H₂/cC₄F₆H₂ | | | 2.0 | 1.5 | 1.1 | 1.5 | 1.5 | 1.5 | 1.5 |
| Conversion rate (mol%) | | | 100.0 | 97.6 | 94.2 | 99.8 | 98.6 | 99.8 | 86.3 |
| Selectivity (mol%) | | | | | | | | | |
| | cC₄F₆H₂ | | 99.6 | 99.0 | 97.7 | 98.9 | 99.7 | 99.4 | 98.2 |
| | | Cis-isomer | 17.7 | 17.1 | 15.9 | 7.2 | 17.5 | 7.4 | 20.9 |
| | | Trans-isomer | 81.9 | 81.9 | 81.8 | 91.7 | 82.2 | 92.0 | 77.3 |
| | Others | | 0.4 | 1.0 | 2.3 | 1.1 | 0.3 | 0.6 | 1.8 |
| | Total | | 100 | 100 | 100 | 100 | 100 | 100 | 100 |

## Claims

1. A method for producing a halogenated cycloalkane compound, comprising:
(IIa) reacting a halogenated cycloalkene compound and a hydrogen-containing gas in the presence of a hydrogenated catalyst to obtain a halogenated cycloalkane compound.

2. The production method according to claim 1, comprising, before step (IIa):
(I) bringing a catalyst into contact with a hydrogen-containing gas to obtain the hydrogenated catalyst.

3. A method for producing a halogenated cycloalkane compound, comprising:
(IIb) reacting a halogenated cycloalkene compound and a hydrogen-containing gas in the presence of a catalyst having a hydrogen content of 0.1 to 1.5 mass% to obtain a halogenated cycloalkane compound.

4. The production method according to claim 3, wherein the catalyst has a specific surface area of 50 to 500 m²/g.

5. The production method according to any one of claims 1 to 4, wherein the halogenated cycloalkane compound is a halogenated cycloalkane compound represented by formula (1): 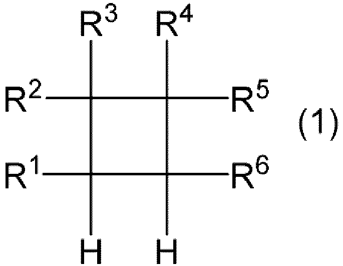 wherein R¹, R², R³, R⁴, R⁵, and R⁶ are the same or different and each is a hydrogen atom, a halogen atom, or a perfluoroalkyl group; when cis-trans isomers are present, both isomers are contained; provided that not all of R¹, R², R³, R⁴, R⁵, and R⁶ are hydrogen atoms.

6. The production method according to any one of claims 1 to 5, wherein the halogenated cycloalkene compound is a halogenated cycloalkene compound represented by formula (2): 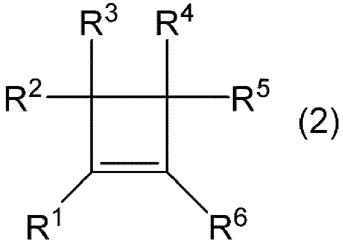 wherein R¹, R², R³, R⁴, R⁵, and R⁶ are the same or different and each is a hydrogen atom, a halogen atom, or a perfluoroalkyl group; when cis-trans isomers are present, both isomers are contained; provided that not all of R¹, R², R³, R⁴, R⁵, and R⁶ are hydrogen atoms.

7. The production method according to any one of claims 1 to 6, wherein the catalyst and hydrogenated catalyst comprise at least one transition metal element belonging to groups 8 to 11 of the periodic table.

8. The production method according to any one of claims 2 and 4 to 7, wherein step (I) is a step of bringing 10 mL to 1000 L of the hydrogen-containing gas into contact with 1 mol of the catalyst.

9. The production method according to any one of claims 2 and 4 to 8, wherein in step (I), the contact time (W/F) of the hydrogen-containing gas with the catalyst is 0.1 to 200 g·sec/cc.

10. A hydrogenated catalyst for use in obtaining a halogenated cycloalkane compound by reacting a halogenated cycloalkene compound and a hydrogen-containing gas, the hydrogenated catalyst having a hydrogen content of 0.1 to 1.5 mass%.

11. The hydrogenated catalyst according to claim 10, comprising at least one transition metal element belonging to groups 8 to 11 of the periodic table.

12. The hydrogenated catalyst according to claim 10 or 11, which has a specific surface area of 50 to 500 m²/g.

13. A composition comprising a halogenated cycloalkane compound represented by formula (1): 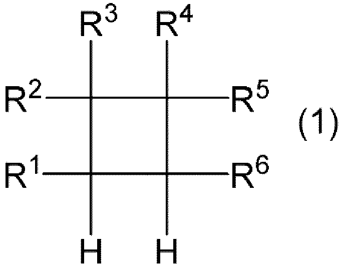 wherein R¹, R², R³, R⁴, R⁵, and R⁶ are the same or different and each is a hydrogen atom, a halogen atom, or a perfluoroalkyl group; when cis-trans isomers are present, both isomers are contained; provided that not all of R¹, R², R³, R⁴, R⁵, and R⁶ are hydrogen atoms,
the halogenated cycloalkane compound represented by formula (1) being contained in an amount of 90.0 to 99.9 mol% based on the total amount of the composition, which is taken as 100 mol%.

14. The composition according to claim 13, which is used as a cleaning gas or an etching gas.
